# EUROPEAN PATENT APPLICATION

(11) **EP 1 658 827 A1**
(43) Date of publication of application: **24.05.2006**
(21) Application number: 05025564.5
(22) Date of filing: 23.11.2005
(51) Int. Cl.: A61F 2/06, A61L 29/04

(54) **Sheath of biaxially oriented polymer**

(30) Priority: 23.11.2004 US 997599
(71) Applicant: Medtronic Vascular, Inc., Santa Rosa, CA 95403 (US)
(72) Inventor: Murray, Robert, Santa Rosa, CA 95409 (US)
(74) Representative: Zimmermann, Gerd Heinrich

(57) **Abstract**

A sheath and method of forming is disclosed for a bi-axial oriented made of high molecular weight polymers. The high molecular weight polymers provide a bi-axial oriented sheath with a high hoop stress and a thin wall thickness. A device and method of use is also disclosed for a device for delivery of a self-expanding prosthesis covered by a bi-axial oriented sheath made of high molecular weight polymers. The bi-axial oriented sheath may be moved from a first position constraining the self-expanding prosthesis to a second position releasing the self-expanding prosthesis. During delivery, the device is inserted into the vascular system of the patient, with the self-expanding prosthesis positioned at the location to be treated. The self-expanding prosthesis is then released by moving the bi-axial oriented sheath from the first to second positions, releasing the self-expanding prosthesis.

## Description

### FIELD OF THE INVENTION

The present invention relates generally to methods and devices for delivering and deploying a self-expanding prosthesis, such as stents, stent-grafts used to treat diseases and conditions of the human vasculature, and more particularly to a bi-axial oriented sheath used to constrain the self-expanding prosthesis.

### BACKGROUND OF THE INVENTION

Self-expanding prosthesis, such as stents, stent-grafts and other structures, are known in the prior art for maintaining the patency of a diseased or weakened vessel or other passageway. They have been implanted in various body passageways such as blood vessels, the urinary tract, the biliary tract, and other body lumens. These self-expanding prosthesis are inserted into the vessel or passageway, positioned across the treatment area and then are allowed to self expand to keep the vessel or passageway open or protect a weakened area, such as an aneurysm. Effectively, the self-expanding prosthesis overcomes the natural tendency of the weakened area of the vessel to close or burst. Stents and stent-grafts used in the vascular system are generally implanted transluminally.

Self-expanding prosthesis may be thought of as mechanically compressed springs which expand when released, and/or they may be constructed from shape-memory materials including shape memory polymers and metals, such a nickel-titanium (nitinol) alloys, and the like which have shape-memory characteristics.

Delivery devices that transport and deliver self-expanding prosthesis usually include an external protective sheath covering the self-expanding prosthesis to prevent premature expansion at body temperatures for heat induced shape memory prosthesis or to contain mechanically restrained or stress induced shape memory prosthesis. Due to the constant expansion pressure of the self-expanding prosthesis against the sheath, the sheath needs a significant amount of radial strength to prevent chronic stretching or polymer creep. This strength is usually obtained by using thick walled tubing made from materials such as PTFE, PEEK and the like. Such thick walled sheaths increase the profile of the delivery device, necessitating use of a delivery catheter with a large diameter. The large diameter of the delivery catheter may in turn increase the risk of complications at the patient access site. The increased profile also detracts from the ability of the delivery device to navigate through tortuous vessels or passageways. The increased cross-sectional profile of the delivery device may make it impossible to deliver a self expanding prosthesis to the treatment area.

Accordingly, it would be desirable to provide a protective sheath having high radial strength properties and a thin wall thickness for a low profile for use on a self-expanding prosthesis delivery device. Furthermore, other desirable features and characteristics according to the present invention will become apparent from the subsequent detailed description and the appended claims, taken in conjunction with the accompanying drawings and the foregoing technical field and background

### SUMMARY OF THE INVENTION

The invention relates to a sheath used for constraining a self-expanding prosthesis. The sheath is bi-axial oriented and made of high molecular weight polymers. The high molecular weight polymers may include Nylon 12, polyether block amide (PEBAX), polyethylene terephthalate (PET), and polyethylene. One of the advantages of the high molecular weight polymers is that they may withstand a high hoop stress with a thin wall thickness.

A method for forming a bi-axial orientation sheath made of high molecular weight polymer is disclosed. The high molecular weight polymers are first extruded forming a parison. The parison is then radially expanded in a mold to form the sheath. The expansion may be done by blow molding, which may include heating the parision and pressurizing the parision tube in the mold. The high molecular weight polymers may include nylon 12, PET, PEBAX or polyethylene.

A device is disclosed for delivering a self-expanding prosthesis covered by a bi-axial oriented sheath in a body vessel. The device includes a shaft with a self-expanding prosthesis positioned proximate a proximal end of the shaft. The bi-axial oriented sheath is made of high molecular weight polymers and covers the elongated catheter shaft and self-expanding prosthesis. The bi-axial oriented sheath may be retracted or moved from a first position constraining the self-expanding prosthesis to a second position releasing the self-expanding prosthesis. The device may further include a handle for moving the sheath from the first position to the second position. The self-expanding prosthesis may be made of nickel-titanium or mechanically compressible spring material. The high molecular weight polymers used for the bi-axial oriented sheath may include nylon 12, PET, PEBAX or polyethylene.

A method is disclosed for delivering of a self-expanding prosthesis covered with a bi-axial oriented sheath. The method includes providing a device having the self-expanding prosthesis constrained by the bi-axial oriented sheath made of high molecular weight polymers. The device is inserted into the vascular system of the patient with the self-expanding prosthesis being positioned in a body vessel at a location to be treated. The self-expanding prosthesis is then released by moving the bi-axial oriented sheath from covering the self-expanding prosthesis.

### BRIEF DESCRIPTION OF THE DRAWINGS

The following drawings are illustrative of particular embodiments according to the invention and therefore do not limit its scope. They are presented to assist in providing a proper understanding of the invention. The drawings are not to scale and are intended for use in conjunction with the explanations in the following detailed descriptions. Like reference numerals denote like elements in the drawings, wherein;

FIG. 1 shows one embodiment of a bi-axial oriented sheath tubular blank as it is processed into a final sheath configuration for use;

FIG. 2 shows one embodiment of a stent delivery device utilizing a bi-axial oriented sheath;

FIG. 3 is a cross-sectional view at A-A of FIG. 2;

FIGs. 4 and 5 illustrate the operation and use of the stent delivery device shown in FIG. 2;

FIG. 6 shows one embodiment of a stent-graft delivery device utilizing a bi-axial oriented sheath; and

FIGs. 7 and 8 illustrate the operation and use of the stent-graft delivery device shown in FIG. 6.

### DETAILED DESCRIPTION

Embodiments according to the present invention will find greatest use in the percutaneous placement of a self-expanding prosthesis, such as endovascular stent-grafts and stents, for the treatment of diseases of the vasculature, particularly aneurysms, stenoses, and the like. Graft structures and stents that may be suitable are described in U.S. Pat. Nos. 5,591,195, 5,683,451, 5,824,041 and 6,533,807, the full disclosure of which is incorporated herein by reference.

The self-expanding prosthesis will be radially compressible, and a cover or sheath will maintain the self-expanding prosthesis under compression in a narrow-diameter configuration while they are being introduced to the body lumen, typically during a surgical cutdown or percutaneous introduction procedures. Placement of the self-expanding prosthesis is done by movement (usually retraction) of the sheath, releasing the self-expanding prosthesis at a target location in the vessel. Many of the self-expanding prosthesis, such as those made of nickel-titanium (nitinol) or compressible spring materials, are capable of exerting much force on the sheath. Due to the constant expansion pressure of the self-expanding prosthesis trying to expand, the sheath needs a significant amount of radial strength to prevent chronic sheath stretching or polymer creep.

Embodiments according to the present invention relate to a bi-axially oriented sheath made from a high molecular weight polymer which when processed, has a high hoop strength resulting in a significantly reduced wall thickness over current sheaths. Suitable high molecular weight polymer materials include Nylon 12, polyether block amide (PEBAX), polyethylene terephthalate (PET), and polyethylene. These high molecular weight polymers can be processed to yield a sheath with high hoop strengths (in excess of 10,000 psi) capable of holding the self-expanding prosthesis in place while only adding 0.001" to 0.008" to the overall profile of the compressed prosthesis OD. The deflection of a tubular body is inversely proportional to the area moment of inertia, which is directly proportional to its wall thickness. Therefore, the amount of deflection for a given load is inversely proportional to its wall thickness. Therefore, a reduced wall thickness will result in lower tracking forces while navigating through tortuous vessels or passageways.

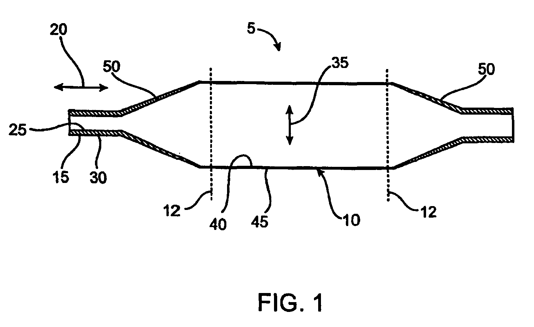

FIG. 1 shows one embodiment of a bi-axial oriented sheath tubular blank 5 as it is processed into a final bi-axial oriented sheath 10 configuration for use (the bi-axial oriented sheath 10 is the portion between dashed lines 12). Any suitable process or method may be used for forming the sheath, such as blow molding a piece of extruded tubing, called a parison, known in the art. The blow molding process for the bi-axial oriented sheath is similar to blow molding a balloon, such as disclosed in US Patent No. 4,490,421 to Levy, incorporated by reference, which discloses a manufacture process for a polymeric balloon used in medical procedures. The fabrication process starts by extruding or providing tubing 15 of high molecular weight polymer material. The tubing is stretched or pulled axially (in the direction identified by arrow 20) from a first length to a second length, which is preferably 1 to 3 times the first length, forming a parison that has a first orientation (axial) and a first internal diameter (ID) 25, which is preferably about one-half a first outer diameter (OD) 30. The parison is then radially expanded (in the direction identified by arrow 35) by expanding means to form the sheathing that has a second ID 40, which is preferably 5 to 8 times the first ID 25. The radial expanding of the tubing is usually done in a mold with pressure by blow molding. By first pulling axially (first orientation) and then expanding radially (second orientation), this process forms a bi-axially oriented sheath 10 with a wall thickness between 0.0005" and 0.004". Once the process is completed, the portions 50 outside to dashed lines 12 are removed to complete the final bi-axial oriented sheath 10 that is ready for use. Depending on the application, a bi-axial oriented sheath 10 may have finished outer diameters from 2.0 to 10.0 mm. A sheath prepared by this process exhibits an unusual combination of film properties, such as toughness, flexibility and tensile strength, with a significant amount of radial strength with thin wall thickness to prevent chronic sheath stretching or polymer creep while constraining the self-expanding prosthesis.

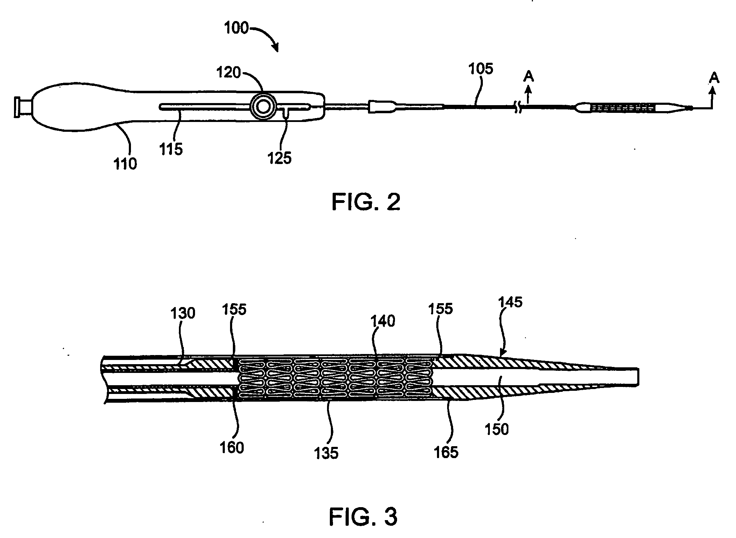

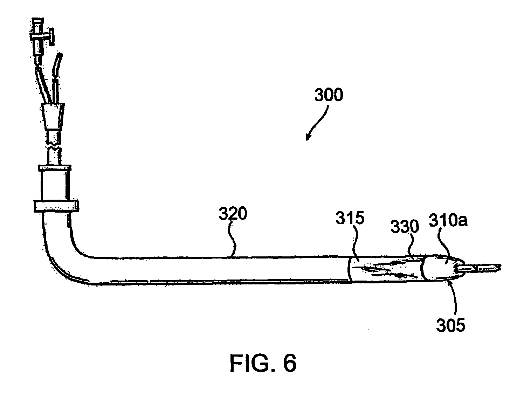

Two types of self-expanding prosthesis are disclosed in the figures. FIGs. 2-5 show one embodiment of a stent delivery device 100 using a bi-axial oriented sheath, and method of use for crossing a lesion and expanding the stenosis. FIGs. 6-8 show one embodiment of a stent-graft delivery device 300 using a bi-axial oriented sheath, and method of use for treating an aneurism. In the embodiments shown in the figures, the bi-axial oriented sheath is shown covering the full length of the catheter including the self-expanding prosthesis and the shaft. In other embodiments, the bi-axial oriented sheath may be joined with another sheath material such that the bi-axial oriented sheath covers the self-expanding prosthesis while the other sheath material covers the shaft.

FIG. 2 shows one embodiment of a stent delivery device 100 for delivering a self-expanding stent to a desired location within a body vessel utilizing a bi-axial oriented sheath suitable for covering and constraining the self-expanding stent. The delivery device 100 includes an elongated member 105 and a handle 110. The handle 110 includes a longitudinal slot 115 along which a knob 120 can move and a transverse slot 125 at the distal end of longitudinal slot 115 in which the knob 120 can rotate.

FIG. 3 is a cross-sectional view at A-A of FIG. 2 showing the elongated member 105 that includes an elongated catheter shaft 130 having a distal portion 145 with a flexible tip and a proximal end near the handle 110. A bi-axial oriented sheath 135 covers the shaft 130 and is moveable with respect to the shaft 130 for releasing the self-expanding stent 140 at the desired treatment site. The bi-axial oriented sheath 135 constrains the self-expanding stent 140 in a compressed state in which the self-expanding stent 140 has a diameter suitable for delivery to a body vessel. Because the self-expanding stent 140 is self-expanding, it is biased radially outward against the interior surface of the bi-axial oriented sheath 135. The bi-axial oriented sheath 135 is preferably made from high molecular weight polymers. The bi-axial oriented sheath 135 inner lumen surface may be coated, such as with silicone, to reduce friction between the shaft 130, self-expanding stent 140 and bi-axial oriented sheath 135 during deployment of the self-expanding stent 140. The self-expanding stent 140 is positioned on the shaft 130 at the distal portion 145 of the elongated member 105 and is preferably made from a shape memory material, such as nitinol or a mechanically compressible spring material. The bi-axial oriented sheath 135 has a substantially constant diameter along the length as shown in FIG. 3, although its distal portion can be enlarged or reduced, depending upon the size of the self-expanding stent 140, to accommodate the stent within the delivery device 100. The shaft 130 may also include a guidewire lumen 150 extending through to the distal portion 145 of the shaft 130. For clarity, the guidewire has been omitted from the figures. The shaft 130 may also include shoulders 155 proximal to the stent 140. The shoulders 155 prevent the stent 140 from moving along the shaft 130 during delivery and also prevent the self-expanding stent 140 from moving with the bi-axial oriented sheath 135 during deployment.

The self-expanding stent 140 is released by moving (retracting) the bi-axial oriented sheath 135 towards the proximal end of the delivery device, i.e., away from the distal portion 145 of the shaft 130. The bi-axial oriented sheath 135 is connected to release knob 120 on the handle 110 such that movement of the knob 120 towards the proximal end of the handle 110 moves the bi-axial oriented sheath 135 in the proximal direction towards the handle 110, uncovering the self-expanding stent 140. The movement of the bi-axial oriented sheath 135 removes the constraining forces on the compressed self-expanding stent 140, thereby allowing it to expand. Other methods of moving a cover sheath from a self-expanding stent may be used and are known to those skilled in the art. One or more radio opaque markers may be used to assist in positioning the stent during delivery. In one embodiment, a first radio opaque marker 160 may be positioned on the shaft 130 at the proximal end of the stent 145 and another radio opaque marker 165 be provided on the sheath 135 at the distal end of the stent 140. Movement of the marker 165 on the sheath past the marker 160 on the shaft is preferably indicative of sufficient movement of the sheath 135 such that the stent 140 is no longer constrained by or within the sheath 135 and has been deployed.

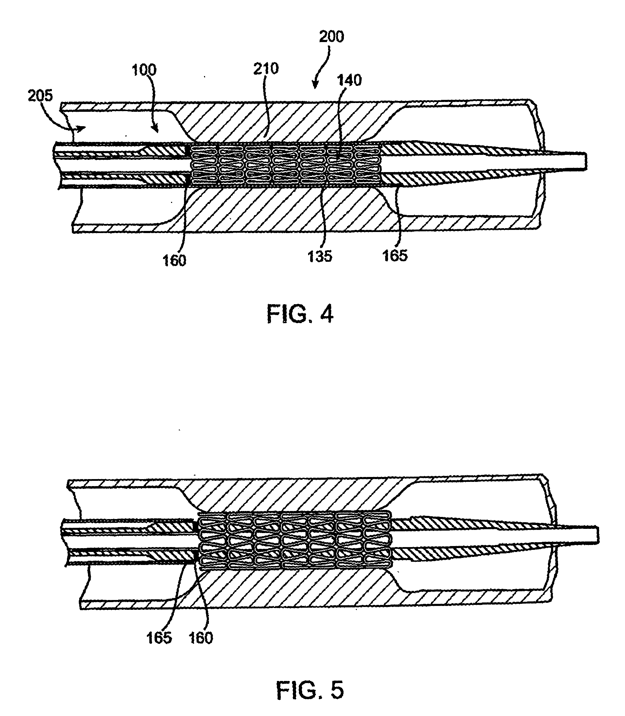

The operation and use of a stent delivery device 100 will now be described. FIG. 4 illustrates the stent delivery device 100 positioned within a patient's vascular system 200. The stent delivery device 100 is initially inserted into vascular system 200, typically through a femoral artery in the groin area, and is advanced through the body vessel 205 until the distal portion 145 of the device is located near the stenosis 210. The stent delivery device 100 is then advanced until the self-expanding stent 140 is positioned across the stenosis 210. The bi-axial oriented sheath 135 is then retracted, releasing the self-expanding stent 140, see FIG. 5. Movement of the marker 165 on the sheath past the marker 160 on the shaft will indicate that the self-expanding stent 140 is no longer constrained by within the bi-axial oriented sheath 135. The self-expanding stent 140 then expands to dilate the stenosis 210. The stent delivery device 100 is then withdrawn from the body vessel 205.

FIGs. 6-8 show one embodiment of a stent-graft delivery device 300 for delivering a self-expanding stent-graft 315 to a desired location within a body vessel utilizing a bi-axial oriented sheath suitable for constraining a self-expanding stent-graft 315. The self-expanding stent-graft 315 is comprised of an expandable material, such as DACRON, with imbedded nitinol springs 330 to provide continuous pressure to a blood vessel wall 332, between 240 and 340 grams of outward pushing force, and simultaneously conform to the specific diameter of a body vessel. An insertion catheter 305 with a balloon 310 is pre-loaded within the self-expanding stent-graft 315, prior to its introduction into the vessel 332. The self-expanding stent-graft 315 is then compressed and loaded within a bi-axial oriented sheath 320 for delivery to the damaged region 335 of the body vessel 332. An inflatable and deflatable graft balloon 310 and an inflatable and deflatable tip balloon 310a, preferably polyurethane balloons, may be disposed about and integral with the distal end of the insertion catheter 305. The bi-axial oriented sheath 320 may be made of high molecular weight polymer materials such as Nylon 12, polyether block amide (PEBAX), polyethylene terephthalate (PET), polyethylene or other similar materials and made as describe above.

The bi-axial oriented sheath 320 is disposed radially about, but not affixed to the self-expanding stent-graft 315, and the self-expanding stent-graft 315 is disposed radially about but not affixed to the insertion catheter 305. After pre-loading, the portion of the insertion catheter having the tip balloon 310a extends outward from the self-expanding stent-graft 315 and the distal end of the bi-axial oriented sheath 320. The tip balloon 310a is inflated as the pre-loaded bi-axial oriented sheath 320 is passed into and through the body vessel 335 toward the damaged region 335.

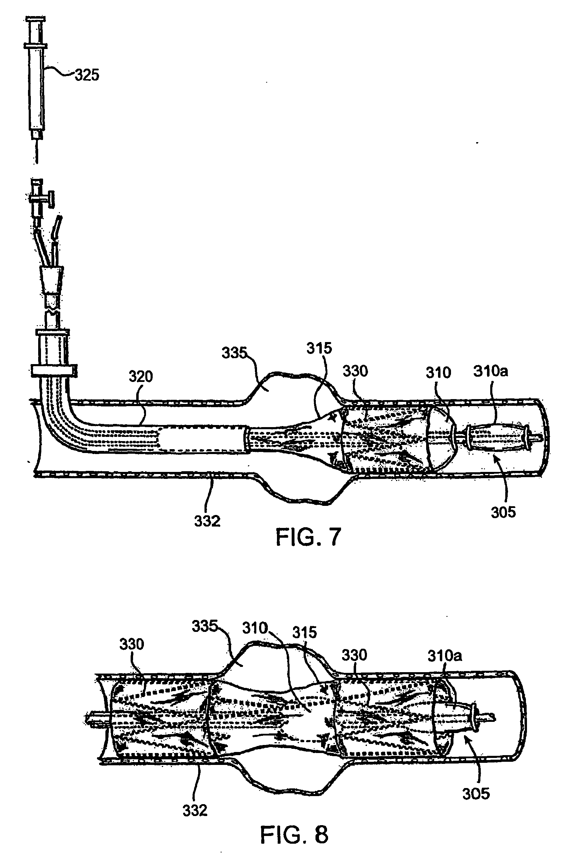

FIGs. 7 and 8 show the stent-graft delivery device 300 in use. The insertion catheter 305 contains a balloon control means for inflation and deflation of stent-graft balloon 310 and tip balloon 310a. The preferred means for inflating balloons 310 and 310a is by injecting a fluid, preferably a radiopaque dye, into balloons 310 and 310a with a syringe 325 through a lumen in the insertion catheter 305. The radiopaque dye provides not only for the inflation of balloons 310 and 310a, but also provides for visual communication enabling the user to determine the location of balloons 310 and 310a relative to positions within stent-graft 315.

As shown further in FIG. 7, the balloon 310 is inflatable to a size consistent with an ability to provide force against the interior of the nitinol spring 330 during its expansion, after its release from the bi-axial oriented sheath introducer 315, thereby providing additional support to the nitinol spring 330 during placement of the self-expanding stent-graft 315 within the body vessel 332 and removal of the bi-axial oriented sheath introducer 320 from its position about the self-expanding stent-graft 315. Additionally, the balloon 310 is inflatable for a duration consistent with the time period necessary to verify that the self-expanding stent-graft 315 is secured in the appropriate position within and against the body vessel wall; a time-period of at least 5 seconds.

As shown further in FIG. 8, after placement is complete, the balloon 310 is deflated slowly to gently introduce blood flow through the self-expanding stent-graft 315 thereby preventing displacement of the self-expanding stent-graft 315 which might be caused by a sudden rush of blood. The partially deflated balloon 310 may be moved throughout the length of the self-expanding stent-graft 315 to unravel and fully open the self-expanding stent-graft 315, and further to smooth out any wrinkles that may have formed in the self-expanding stent-graft 315 during placement. Insertion catheter 305 may then be removed and if the self-expanding stent-graft 315 is appropriately positioned, incisions may be closed.

According to an embodiment of the present invention, a method for delivering of a self-expanding prosthesis is presented. The method comprises inserting a device having a self-expanding prosthesis constrained by a bi-axial oriented sheath made of high molecular weight polymer into a vascular system of a patient; positioning the self-expanding prosthesis in a body vessel proximal to an area to be treated; and releasing the self-expanding prosthesis by moving the bi-axial oriented sheath. Typically, the bi-axial oriented sheath is movable between a first position constraining the self-expanding prosthesis to a second position releasing the self-expanding prosthesis, and releasing the self-expanding prosthesis includes moving the bi-axial oriented sheath from the first position to the second position

While exemplary embodiments have been presented in the foregoing detailed description, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient roadmap for implementing an exemplary embodiment of the invention, it being understood that various changes may be made in the function and arrangement of elements described in an exemplary embodiment without departing from the scope of the invention as set forth in the appended claims.

## Claims

1. A sheath for constraining a self-expanding prosthesis, wherein the sheath comprises bi-axially oriented, high molecular weight polymer.

2. The sheath of claim 1, wherein the high molecular weight polymer is one or more polymers selected from the group consisting of: nylon 12, PET, PEBAX, polyethylene.

3. The sheath of claim 1 or 2, wherein the bi-axially oriented, high molecular weight polymer has a hoop stress greater than 10,000 psi.

4. The sheath of any of claims 1 to 3, wherein the sheath has a diameter between 2.0mm and 10.0mm.

5. The sheath of any of claims 1 to 4, wherein the sheath has a wall thickness between 0.0005" and 0.004".

6. A method for forming a sheath having bi-axial orientation for constraining a self-expanding prosthesis, the method comprising:
extruding tubing of high molecular weight polymer forming a parison having a first orientation, a first inner diameter (ID), a first outer diameter (OD) and a first wall thickness; and
radially expanding the parison in a mold forming a sheath having a second orientation in addition to the first orientation, a second ID, a second OD and a second wall thickness.

7. The method of claim 6, wherein the first orientation is an axial orientation.

8. The method of claim 6 or 7, wherein the second orientation is a radial orientation.

9. The method of any of claims 6 to 8, wherein radially expanding comprises blow molding the parison.

10. The method of claim 9, wherein blow molding the parison comprises heating and pressurizing the parison.

11. The method of any of claims 6 to 10, wherein the high molecular weight polymer is one or more polymers selected from the group consisting of: nylon 12, PET, PEBAX, polyethylene.

12. The method of any of claims 6 to 11, wherein the second ID is 5 to 8 times the first ID.

13. The method of any of claims 6 to 12, wherein the sheath has a hoop stress greater than 10,000 psi.

14. The method of any of claims 6 to 13, wherein the second OD is 2.0 to 10.0 mm.

15. The method of any of claims 6 to 14, wherein the second wall thickness is between 0.0005" to 0.004".

16. A device for delivering a self-expanding prosthesis, said device comprising:
a shaft having a distal end and a proximal end;
a self-expanding prosthesis positioned proximate the proximal end of the shaft; and
a bi-axial oriented sheath made of high molecular weight polymer, the bi-axial oriented sheath configured to cover the self-expanding prosthesis, the bi-axial oriented sheath being movable between a first position constraining the self-expanding prosthesis to a second position releasing the self-expanding prosthesis.

17. The device of claim 16, further comprising a handle at the proximal end of the shaft, the handle having a sheath moving mechanism configured to move the bi-axial oriented sheath from the first position to the second position.

18. The device of claim 16 or 17, wherein the shaft further includes a flexible tip at the distal end.

19. The device of any of claims 16 to 18, wherein the self-expanding prosthesis is made of nickel-titanium.

20. The device of any of claims 16 to 19, wherein the self-expanding prosthesis is made of mechanically compressible spring material.

21. The device of any of claims 16 to 20, wherein the high molecular weight polymer is one or more polymers selected from the group consisting of: nylon 12, PET, PEBAX, polyethylene.

22. The device of any of claims 16 to 21, wherein the bi-axial oriented sheath has a diameter range of 2.0 to 10.0 mm.

23. The device of any of claims 16 to 22, wherein the bi-axial oriented sheath has a wall thickness range of 0.0005" to 0.004".
